# EUROPEAN PATENT SPECIFICATION

(11) **EP 4 061 328 B1**
(45) Date of publication and mention of the grant of the patent: **03.01.2024**
(21) Application number: 20808117.4
(22) Date of filing: 20.11.2020
(51) Int. Cl.: A61K 9/00, A61K 9/20, A61K 31/423

(54) **ZONISAMIDE ORODISPERSIBLE TABLETS**
ORODISPERGIERBARE ZONISAMIDTABLETTEN
COMPRIMÉS DE ZONISAMIDE ORODISPERSIBLES

(30) Priority: 22.11.2019 EP 19383034
(43) Date of publication of application: 28.09.2022
(73) Proprietor: BIOHORM, S.L., 08184 Palau-solità i Plegamans (Barcelona) (ES)
(72) Inventor: STRUSI, Orazio Luca, 08206 Sabadell - Barcelona (ES); CODERCH, Montserrat, 08172 Sant Cugat del Vallès (ES)
(74) Representative: ABG Intellectual Property Law, S.L.
(86) International application number: PCT/EP2020/082809
(87) International publication number: WO 2021/099531

(56) References cited:
- EP-A1- 2 251 005

## Description

### FIELD OF THE INVENTION

The present invention relates to solid pharmaceutical formulations comprising zonisamide as an active ingredient, in particular it relates to a tablet for oral administration of zonisamide which disintegrates rapidly by the action of saliva in the oral cavity, having also good palatability, good mechanical strength properties and obtainable by conventional manufacturing methods.

### BACKGROUND OF THE INVENTION

Zonisamide, 1,2-benzisoxazole-3-methane sulphonamide, is a drug displaying anti-convulsing effects, being useful in the treatment of the symptoms of epilepsy, as well as an adjuvant to levodopa in the treatment of Parkinson's disease. In Europe, for example, it is marketed under the brand name "Zonegran^{®}", although some generic versions exist, and can be found formulated as capsules and orodispersible tablets. In the form of orodispersible tablets, it is only marketed in Japan under the brand name "Trerief^{®}".

Orodispersible tablets (ODTs) are defined in the European Pharmacopoeia (Ph.Eur.) 8th edition, published in July 2013, as non-coated tablets for placing in the mouth which disintegrate quickly before they are swallowed. It also establishes 3 minutes as the time under which they must disintegrate in the disintegration test for tablets and capsules, according to the Ph. Eur. 2.9.1. method.

The development of solid formulations that disintegrate quickly in the mouth without requiring water is of interest due to the advantages that these pharmaceutical formulations provide for patients who have difficulty in swallowing, such as old people, infants, patients with mental problems and non-cooperative patients, as well as the population in general; since it makes it possible for the drug to be administered without the need for water.

Several technologies are available to produce commercial ODTs. The technologies are usually grouped according to the method employed in the preparation: freeze drying (Zydis^{®}, Quicksolv^{®} and Lyoc^{®}), molding (FlashDose^{®}) and compaction technologies.

Towa Pharmaceutical Co. Ltd has developed a technology for producing ODT products called RACTBAB. Tablets manufactured using this technology are said to be resistant against humidity, to show good friability and to dissolve easily in the oral cavity.

The RACTAB technology consists in manufacturing so-called rapidly dissolving granules (RDGs) by fluid bed, spray coating a suspension of saccharide with crospovidone and corn starch. The granules obtained are characterized by a narrow particle distribution, large surface area and micropores. In order to guarantee a tablet with acceptable hardness the RDGs are blended further with micronized ethylcellulose, which in this case is used as binder agent, for improving the physical strength and the resistance against humidity.

Despite the acceptable properties concerning hardness and water absorption of the tablets obtained with this technology, the manufacturing method requires a multi-step fluid bed granulation process which is not only energy consuming but also represents a long and time consuming process. Additionally, the use of several excipients, among them micronized ethylcellulose, which is not standard ODT material, increases the costs of the RACTAB technology even further.

Each ODT technology has its own advantages and disadvantages, however one of the key aspects that must be balanced is to achieve adequate tablet mechanical properties while maintaining short disintegration times.

In this respect, while ODTs prepared using freeze drying (lyophilization) technology display quick disintegration profiles, they exhibit high friability, low stability at high temperatures and humidity levels, and show poor mechanical properties. Furthermore, this technology relies on expensive machinery, wherein the cost can be further expanded due to special packing required sometimes, and can be troublesome when scaling up.

The molding technology can be based in two different processes, the solvent method and the heat method, and allows for the preparation of high drug dose tablets and for the resulting tablets presenting a rapid dissolution. On the other hand, the so prepared tablets usually exhibit low mechanical resistance, and the resulting tablets are subject to erosion and breakage during the handling and opening of the blister pockets.

Compaction technologies are the conventional processes used to prepare orodispersible tablets. Two common approaches exist, the first one being based on granulation (wet granulation, dry granulation and mold granulation) followed by compression; and the second one based on the direct compression of the powdery mixture. This technology has some advantages, since it is a well-established technology, thus having low manufacturing costs and allows easy technology transfer (e.g. easy to transfer to different producers). A key feature of this technology in the manufacture of ODTs is the use of the so called superdisintegrants which accelerate the rate of disintegration and hence dissolution, which can be further aided by water soluble excipients and, sometimes, effervescent agents. However, despite the economic benefits and simpler technological requirements, usually, the ODTs prepared using this method, have higher disintegration times than those prepared using other technologies as a result of the higher mechanical resistance of the tablets prepared using this technology, since these two parameters, usually, have an inverse correlation.

Orodispersible tablets can be useful in various applications, in particular in cases where the patients are elderly persons or children having problems to swallow the normal tablets or capsules. For example, patients suffering from Parkinson disorder have an effected motor system so that an orodispersible tablet is advantageous.

Dainippon developed and commercializes oral dispersible tablets containing zonisamide under the brand Trerief^{®} OD. The Trerief^{®} OD composition comprises D-mannitol, corn starch, crystalline cellulose, ethyl cellulose, polyvinyl alcohol (partial saponification), talc, light anhydrous silicic acid, aspartame (L-phenylalanine compound), magnesium stearate, yellow ferric oxide.

As in the case of RACTAB technology, the Trerief^{®} OD formulation contains crystalline cellulose and ethyl cellulose, both used as binder, excipients that are not standard ODT excipients. These two excipients are required in Trerief^{®} for increasing the tablet's hardness in order to arrive at acceptable levels. However, the hardness test of 50 mg Trerief OD tablets result in values of approximately 114N which is not sufficient for ensuring the integrity of the tablets, in particular if the tablets are to be shipped in bulk. In particular, when the orodispersible tablets are stored, hardness will decrease making them more sensitive to breakage. Patent document EP2251005 discloses rapidly disintegrating zonisamide tablets.

Therefore, it is an object of this invention to provide orodispersible tablets comprising zonisamide having a high hardness (conferring them a good mechanical resistance), while maintaining a quick disintegration desirable for ODT, and which can be obtained by simple and economic processes, such as direct compression. In particular, it is one object of the invention to provide orodispersible tablets comprising zonisamide having an improved mechanical resistance that allows to maintain an excellent to good hardness over time.

### BRIEF SUMMARY OF THE INVENTION

The authors of the present invention have surprisingly found that a tablet prepared by direct compression comprising zonisamide; particles comprising mannitol, microcrystalline cellulose, carmellose and crospovidone; a lubricant and optionally one or more sweeteners, has a high hardness and, despite this high hardness, maintains good to excellent disintegration times, which involves important advantages with respect to other more complex technologies which require preparing tablets with low resistance and high porosity in order to get short disintegration times. The high hardness is such that it will protect tablets when shipped in bulk. Furthermore, the tablets according to the present invention show excellent stability that allows maintaining a sufficient hardness over time, while still showing good to excellent disintegration times.

Direct compression provides important advantages over other complex techniques since the active ingredient is not subjected to humidity conditions (water or other solvents) or to high temperatures, conditions which are known to diminish the stability of the oral formulation. In addition, due to its simplicity, it only requires simple machinery leading to less economic and energetic manufacturing costs.

Therefore, in a first aspect, the present invention relates to an orally disintegrating tablet comprising:
(i) Zonisamide;
(ii) particles comprising:
   a) a sugar or a sugar alcohol, such as mannitol, erythritol, sorbitol, D-glucitol (maltitol), trehalose, lactose, maltose, glucose, fructose, sucrose, mannose, or a combination thereof;
   b) a cellulose, such as crystalline cellulose, microcrystalline cellulose and powdered cellulose;
   c) an acid-type carboxymethylcellulose as the first disintegrant component, such as carmellose; and
   d) a second disintegrant component, which is not an acid-type carboxymethylcellulose, such as crospovidone, sodium croscarmellose, sodium carboxymethyl starch, low substituted hydroxypropylcellulose, calcium carboxymethylcellulose, hydroxypropyl starch, starch sodium glycolate, and starch;
(iii) a lubricant; and
(iv) optionally one or more sweeteners and and/or other pharmaceutically acceptable excipients.

A second aspect of the present invention relates to a process for the preparation of a tablet as defined above, which comprises:
a) preparing a mixture comprising the ingredients (i), (ii), (iii) and (iv), and;
b) applying direct compression to the mixture obtained in step a), preferably using a pressure between 5 and 50 kN.

A third aspect of the invention refers to orally disintegrating tablets prepared by direct compression, according to first and second aspect, packaged into a polymer film, preferably made of polyvinyl chloride, polypropylene or polyvinylidene dichloride and an aluminium seal.

### DETAILED DESCRIPTION OF THE INVENTION

The present invention is directed to an orally disintegrating tablet obtainable by direct compression of a mixture, said mixture comprising:
(i) zonisamide;
(ii) particles comprising
   a) a sugar or a sugar alcohol, such as mannitol, erythritol, sorbitol, D-glucitol (maltitol), trehalose, lactose, maltose, glucose, fructose, sucrose, mannose, or a combination thereof;
   b) a cellulose, such as crystalline cellulose, microcrystalline cellulose and powdered cellulose;
   c) an acid-type carboxymethylcellulose as the first disintegrant component, such as carmellose; and
   d) a second disintegrant component, which is not an acid-type carboxymethylcellulose, such as crospovidone, sodium croscarmellose, sodium carboxymethyl starch, low substituted hydroxypropylcellulose, calcium carboxymethylcellulose, hydroxypropyl starch, starch sodium glycolate, and starch;
(iii) a lubricant; and
(iv) optionally one or more sweeteners and and/or other pharmaceutically acceptable excipients.

In a preferred embodiment of the first aspect, the present invention relates to an orally disintegrating tablet comprising:
(i) zonisamide;
(ii) particles comprising,
   a) mannitol;
   b) microcrystalline cellulose;
   c) carmellose;
   d) crospovidone
(iii) a lubricant;
(iv) optionally, one or more sweeteners.

The orodispersible tablet is advantageously used in cases where administration without water is necessary, cases of administration to patients who have difficulty in swallowing tablets, or cases of administration to the elderly or to children where there is a fear of blocking the throat if it is unusual tablet form. The orodispersible tablets can be safely administered orally to humans.

The active ingredient of the tablet according to the present invention comprises zonisamide or a pharmaceutical acceptable salt thereof.

The amount of the active ingredient used can vary greatly. Those of ordinary skill in the art will appreciate that the physical characteristics of the active ingredient, the size of the tablet and the requirements of other ingredients will directly influence its limiting content in the formulation. However, in an embodiment of the invention the tablets comprise from 1 to about 20% by weight of zonisamide, preferably from 5-18 % by weight, more preferably from 8-15 % by weight and even more preferably from 10-14 % by weight, in other words, wherein the percentage by weight is relative to the total weight of the composition. In a particularly preferred embodiment, the tablets comprise about 12.5% of zonisamide, based on the total weight of the composition

In absolute amounts, an orodispersible tablet containing zonisamide may comprise said zonisamide in an amount of from 10-500 mg, preferably from 25-200 mg, more preferably in amounts of 25 mg, 50 mg, 100 mg, 150 mg and/or 200 mg.

In an embodiment the orally disintegrating tablets comprise particles of zonisamide having a particle size distribution characterised by a D(97) in the range of 30 µm to 300 µm measured by volume, preferably a D(97) of from 50 µm to 250 µm measured by volume, more preferably D(97) equal or less than 200 µm measured by volume, even more preferably a D(97) equal or less than 150 µm measured by volume.

In an embodiment the orally disintegrating tablets comprise particles of zonisamide having a particle size distribution characterized by a D(90) in the range of from 30 µm to 100 µm measured by volume, preferably D(90) of from 50 µm to 75 µm measured by volume, more preferably D(90) equal or not less than 68 µm measured by volume, even more preferably D(90) equal or not less than 48 µm measured by volume.

In an embodiment the orally disintegrating tablets comprise particles of zonisamide having a particle size distribution characterised by a D(97) in the range of 30 µm to 300 µm measured by volume and a D(90) of from 30 µm to 100 µm measured by volume.

In an embodiment the orally disintegrating tablets comprise particles of zonisamide having a particle size distribution characterized by a D(97) equal or less than 200 µm measured by volume, preferably a D(97) equal or less than 150 µm measured by volume, and a D(90) equal or not less than 48 µm measured by volume, preferably a D(90) equal or not less than 68 µm measured by volume, since mechanical properties such as flowability, compressibility and uniformity of the tablets are improved.

The term flowability as used herein refers to the ability of divided solids (for example powders and granules) to flow vertically under defined conditions. As stated in European Pharmacopeia 8.5 (2.9.16, monograph) the flowability is determined by measuring the time needed for a test sample to flow out of a funnel, which can have different angle and/or orifice diameter according to the flow properties of the material to be tested. Typical orifice diameters of the funnel are 10 mm, 15 mm and 25 mm. The flowability is expressed in seconds and tenths of seconds, related to 100 g of sample.

The term particle size distribution (PSD) refers to the relative percentages by volume of each of the different size fractions of a particulate matter. The particle size distributions can be measured using laser light diffraction equipment, such as Malvern Mastersizer^{®} 2000. Particle size is determined by measuring the angular distribution of laser light scattered by a homogeneous suspension of particles. The size distribution is determined from the light scattering data using the theory of light scattering developed by Gustav Mie. Other types of equipment are also suitable to determine particle size distribution. The term D(n), as used herein, means that n % of the particles in a composition (based on volume) have a diameter equal to or below a specified D value. Therefore, D(97) means that 97% of the particles have a diameter equal or below the specified D value.

The tablet according to the present invention comprises particles comprising a) a sugar or a sugar alcohol, preferably mannitol, b) a cellulose, preferably microcrystalline cellulose, c) an acid-type carboxymethylcellulose as the first disintegrant component, preferably carmellose and d) a second disintegrant component, which is not an acid-type carboxymethylcellulose, preferably crospovidone.

In the context of the present invention, suitable sugars or a sugar alcohols for the particles, comprise mannitol, erythritol, sorbitol, D-glucitol (maltitol), trehalose, lactose, maltose, glucose, fructose, sucrose, mannose, or a combination thereof, preferably mannitol.

In the context of the present invention, suitable celluloses for the particles, comprise crystalline cellulose, microcrystalline cellulose and powdered cellulose, or a combination thereof, preferably microcrystalline cellulose.

In the context of the present invention, a suitable acid-type carboxymethylcellulose as the first disintegrant component for the particles is carmellose.

In the context of the present invention, a suitable second disintegrant component for the particles which is not an acid-type carboxymethylcellulose comprises crospovidone, sodium croscarmellose, sodium carboxymethyl starch, low substituted hydroxypropylcellulose, calcium carboxymethylcellulose, hydroxypropyl starch, starch, sodium glycolate, starch, or a combination thereof, preferably crospovidone.

In the context of the present invention when a numerical value is preceded by the word "about" it is to be understood that said value may vary by ± 5%, the specific value being specifically included. Thus "about 100" should be construed as equivalent to "from 95 to 105, preferably 100".

In an embodiment the tablet according to the present invention, comprises particles comprising a) a sugar or a sugar alcohol, preferably mannitol, b) a cellulose, preferably microcrystalline cellulose, c) an acid-type carboxymethylcellulose as the first disintegrant component, preferably carmellose and d) a second disintegrant component, which is not an acid-type carboxymethylcellulose, preferably crospovidone, wherein the particles are present in an amount from 80% to 90% by weight, preferably from 82% to 88% by weight, more preferably from 84-86.5 % by weight of the tablet.

In an embodiment the particles comprise a sugar or a sugar alcohol, preferably mannitol from 40% to 60% by weight of the particles, preferably from 45% to 60% by weight of the particles, more preferably from 53-59 % by weight of the particles.

In an embodiment the particles comprise a cellulose, preferably microcrystalline cellulose from 10% to 30% by weight of the particles, preferably from 15% to 25% by weight of the particles, more preferably from 17% to 23% by weight of the particles.

In an embodiment the particles comprise an acid-type carboxymethylcellulose as the first disintegrant component, preferably carmellose from 10% to 30% by weight of the particles, preferably from 12% to 25% by weight of the particles, more preferably from 17% to 23% by weight of the particles.

In an embodiment the particles comprise a second disintegrant component, which is not an acid-type carboxymethylcellulose, preferably crospovidone from 1% to 7% by weight of the particles, preferably from 2% to 5% by weight of the particles, more preferably from 3.4-4.6 % by weight of the particles.

In an embodiment the particles comprising a) a sugar or a sugar alcohol, preferably mannitol, b) a cellulose, preferably microcrystalline cellulose, c) an acid-type carboxymethylcellulose as the first disintegrant component, preferably carmellose and d) a second disintegrant component, which is not an acid-type carboxymethylcellulose, preferably crospovidone have an average particle size between 50 µm and 200 µm, more preferably between 90 µm and 150 µm, more preferably between 95 µm and 150 µm, more preferably between 100 µm and 150 µm, more preferably between 120 µm and 150 µm, even more preferably between 139 µm and 141µm, and most preferably about 140 µm, measured by volume.

In an embodiment, the particles containing the four components, a), b), c) and d) may be produced by any method known to the skilled person, such as for example a wet granulation. Said method may comprise a single granulation process, or a two-step granulation process, such as the ones described in patent application EP2898899 A1.

In a single granulation process, particle components a), b), c) and d) are charged into a fluidized-bed granulator with subsequent spraying with water thereby obtaining the granules.

In a two-step granulation process, particle components a), c) and d), are charged into a fluidized-bed granulator with subsequent spraying with water, thus resulting in a first granule. Said first granule is further mixed with particle component b), to obtain the second granule comprising particle components a), b), c) and d).

In an embodiment the particles can be acquired from Daicel under the tradenames Granfiller^{®}-D211 or Granfiller^{®}-D215.

The lubricant is used herein as an additional excipient. Suitable examples of lubricants include but are not limited to talc, sodium benzoate, sodium stearyl fumarate (e.g. Pruv, Lubripharm SSF), calcium stearate, magnesium stearate, zinc stearate, glyceryl behenate, stearic acid and glyceryl monostearate. Preferably the lubricants are selected from the group comprising sodium stearyl fumarate (Pruv), magnesium stearate or a combination thereof, more preferably the lubricant is sodium stearyl fumarate (Pruv). It has been found that use of sodium stearyl fumarate may yield in ODT tablets having an increased hardness while maintaining an excellent disintegration time.

In a preferred embodiment the lubricant(s) is (are) used in an amount of 0.1 % to 5 % by weight of the tablet, preferably from 0.5-4.5 % by weight, and more preferably from 1-4 % by weight.

Optionally, the tablets of the present invention may comprise one or more sweeteners, wherein the sweeteners may be selected from the group comprising aspartame, potassium acesulfame, sodium saccharinate, neohesperidine dihydrochalcone, sucralose, sucrose, fructose, monoammonium glycyrrhizinate, and mixtures thereof. Preferably the sweetener is aspartame. Preferably the sweetener of the present invention is used in an amount of about 0.1 to 7 % by weight of the tablet, preferably from 1-5 % by weight, more preferably from 1-3 % by weight, and even more preferably from 1.5-2.5 % by weight.

The tablet formulation of the present invention can also include other pharmaceutically acceptable excipients like surfactants, flavouring agents, lubricants, sweeteners, glidants, anti-adherents and mixtures thereof, which affect the elegancy and performance of the orodispersible pharmaceutical compositions. The additional pharmaceutically acceptable excipients used in said formulation are present in small amounts, e.g. generally less than 10 %, preferably 5 % of the total weight of the tablet.

Suitable flavouring agents used in the composition of the present invention include but are not limited to strawberry, cherry, orange, peppermint, black currant, banana, raspberry, red fruits, wild berries and caramel flavour. Preferably the flavouring agents of the present invention are used in an amount of less than 5 % by weight of the tablet, preferably less than 4 % by weight, more preferably less than 2 % by weight, even more preferably less than 1%.

In an embodiment of the first aspect, the present invention relates to an orally disintegrating tablet comprising:
(i) zonisamide;
(ii) particles comprising
   a) a sugar or a sugar alcohol, such as mannitol, erythritol, sorbitol, D-glucitol (maltitol), trehalose, lactose, maltose, glucose, fructose, sucrose, mannose, or a combination thereof;
   b) a cellulose, such as crystalline cellulose, microcrystalline cellulose and powdered cellulose;
   c) an acid-type carboxymethylcellulose as the first disintegrant component, such as carmellose; and
   d) a second disintegrant component, which is not an acid-type carboxymethylcellulose, such as crospovidone, sodium croscarmellose, sodium carboxymethyl starch, low substituted hydroxypropylcellulose, calcium carboxymethylcellulose, hydroxypropyl starch, starch sodium glycolate, and starch;
(iii) a lubricant; and
(iv) optionally one or more sweeteners and and/or other pharmaceutically acceptable excipients
wherein zonisamide (i) have a particle size distribution characterized by a D(97) in the range of 30 µm to 300 µm measured by volume.

In a preferred embodiment of the first aspect, the present invention relates to an orally disintegrating tablet comprising:
(i) zonisamide;
(ii) particles comprising,
   a) mannitol;
   b) microcrystalline cellulose;
   c) carmellose;
   d) crospovidone
(iii) a lubricant;
(iv) optionally, one or more sweeteners.
wherein zonisamide (i) has a particle size distribution characterized by a D(97) in the range of from 30 µm to 300 µm measured by volume, preferably a D(97) equal or less than 200 µm measured by volume, more preferably D(97) equal or less than 150 µm measured by volume.

In a more preferred embodiment of the first aspect, the present invention relates to an orally disintegrating tablet comprising:
(i) zonisamide;
(ii) particles comprising,
   a) mannitol;
   b) microcrystalline cellulose;
   c) carmellose;
   d) crospovidone
(iii) a lubricant;
(iv) optionally, one or more sweeteners.
wherein zonisamide (i) has a particle size distribution characterized by a D(97) in the range from 30 µm to 300 µm measured by volume and by a D(90) in the range from 30 µm to 100 µm measured by volume.

In an embodiment of the first aspect, the present invention relates to an orally disintegrating tablet comprising:
(i) from 1 to about 20% by weight zonisamide;
(ii) from 80% to about 90% by weight of particles comprising
   a) a sugar or a sugar alcohol, such as mannitol, erythritol, sorbitol, D-glucitol (maltitol), trehalose, lactose, maltose, glucose, fructose, sucrose, mannose, or a combination thereof;
   b) a cellulose, such as crystalline cellulose, microcrystalline cellulose and powdered cellulose;
   c) an acid-type carboxymethylcellulose as the first disintegrant component, such as carmellose; and
   d) a second disintegrant component, which is not an acid-type carboxymethylcellulose, such as crospovidone, sodium croscarmellose, sodium carboxymethyl starch, low substituted hydroxypropylcellulose, calcium carboxymethylcellulose, hydroxypropyl starch, starch sodium glycolate, and starch;
(iii) from 0.1 to 5% of a lubricant; and
(iv) optionally from 0.1% to 7% of one or more sweeteners and and/or other pharmaceutically acceptable excipients
wherein the percentage by weight is relative to the total weight of the composition and wherein zonisamide particles (i) have a particle size distribution characterized by a D(97) in the range from 30 µm to 300 µm measured by volume, preferably a D(97) equal or less than 200 µm measured by volume, more preferably D(97) equal or less than 150 µm measured by volume.

The formulation used in the invention allows preparing orodispersible tablets with high hardness while maintaining good disintegration times. The high hardness further protects the tablets when shipped in bulk. It has also been found that upon storage under unpacked conditions, at 25 °C and 75% relative humidity (RH) or at 40°C and 75% RH, the tablets of the present invention maintain more than 50% of their initial hardness after 15 days of storage, whereas the commercially available tablets Trerief^{®} lose more than 50% of their initial hardness. Moreover, unpackaged orally disintegrating tablets of the present invention maintain more than 50% of their initial hardness upon storage conditions at 25 °C / 75% RH for at least 3 months.

As used herein, tablet hardness is defined by the mechanical resistance to external stimuli and is measured by the force, applied *via* diametric compression, required to break, or induce fractures in a tablet. In general, the method for testing tablet hardness according to Ph. Eur. 2.9.8 is followed. Normally, 10 samples are used as required. However, depending on the batch size manufactured, and mostly at laboratory scale, the number of samples may also be 5 or even 3.

As used herein, tablets containing 50 mg of zonisamide having high hardness, are tablets having a hardness above 100 N. In a preferred embodiment the tablets containing 50 mg of zonisamide of the present invention have a tablet hardness from 100 N to 300 N, preferably from 120 N to 250 N, more preferably from 125 N to 250 N, even more preferably from 130 N to 200 N, even more preferably from 140 N to 180 N, and even more preferably from 150 N to 180 N.

As used herein, tablets containing 25 mg of zonisamide having high hardness, are tablets having a hardness of 80 N or more. In a preferred embodiment the tablets containing 25 mg of zonisamide of the present invention have a tablet hardness from 80 N to 200 N, preferably from 80 N to 180 N, more preferably from 80 N to 160 N, even more preferably from 85 N to 150 N, and even more preferably from 90 N to 140 N.

As used herein, disintegration time is the time required for a tablet to completely disintegrate in water. In the present specification, two disintegration protocols are applied. Disintegration protocol I requires that an ODT tablet is placed in a glass beaker having 65 mm of diameter, containing 40 mL of purified water, at 37.5°C. The time of the onset of disintegration (i = initial), and the time of complete disintegration are recorded. The disintegration time is the time when the tablet completely disintegrates. Disintegration protocol II requires that an ODT tablet according to the present invention is placed in a disintegration apparatus of the type Tablet Disintegration Tester DTG 1000 of Copley Scientific filled with 800 mL of purified water, at 37.5 °C. The disintegration time is the time when the tablet completely disintegrates. As used herein a tablet having a good disintegration time is a tablet which completely disintegrates in less than 45 seconds, either according to Disintegration protocol I or to Disintegration protocol II. Preferably, Disintegration protocol II is used.

In a preferred embodiment, the tablets of the present invention disintegrate in 30 seconds or less, more preferably in 27 seconds or less, more preferably in 25 seconds or less, more preferably in 23 seconds or less, even more preferably in 20 seconds or less, and even more preferably in less than 20 seconds, when measured according to Disintegration protocol II. Surprisingly, the tablets of the present invention show a faster disintegration times than the commercially available tablets despite the fact that the tablets of the present invention show higher hardness.

In a particular embodiment, the orally disintegrating tablet of the present invention have, when 50 mg of zonisamide are used, a hardness of more than 100 N and a disintegration time, measured according to Disintegration protocol II, of less than 30 seconds, preferably a hardness of more than 120 N and a disintegration time, measured according to Disintegration protocol II of less than 27 seconds, more preferably a hardness of more than 125 N and a disintegration time, measured according to Disintegration protocol II, of less than 25 seconds, and even more preferably a hardness of more than 125 N and a disintegration time, measured according to Disintegration protocol II, of less than 23 seconds.

In a particular embodiment, the orally disintegrating tablet of the present invention have, when 25 mg of zonisamide are used, a hardness of more than 80 N and a disintegration time, measured according to Disintegration protocol II, of less than 30 seconds, preferably a hardness of more than 80 N and a disintegration time, measured according to Disintegration protocol II, of less than 27 seconds, more preferably a hardness of more than 85 N and a disintegration time, measured according to Disintegration protocol II, of less than 27 seconds, and even more preferably a hardness of more than 85 N and a disintegration time, measured according to Disintegration protocol II, of less than 25 seconds.

The orally disintegrating tablet of the present invention is prepared by direct compression of a dry powdered mixture. The term "*direct compression*" is used in the context of the invention to define a process by which tablets are compressed directly from powder blends of the active ingredient and the excipients (including the particles comprising mannitol, microcrystalline cellulose, carmellose and crospovidone, diluents, fillers, disintegrants and lubricants), which flow uniformly into a die cavity and form a firm compact.

By the term "dry powdered mixture" it is understood a mixture of ingredients in powder form, wherein said ingredients may have been previously and independently passed through a sieve with mesh size lower than 650 µm, preferably less than 300 µm, without having been subjected to any granulation process, dissolution or dispersion in a liquid medium. In a preferred aspect, during manufacturing of the tablets only the active ingredient zonisamide is passed through a sieve. In another aspect, sieving is carried out using sieves with different mesh sizes that may be used for each ingredient of the dry powdered mixture, and/or for combinations thereof with the condition that all the sieves have a mesh size lower than 650 µm, preferably less than 300 µm, without having been subjected to any granulation process, dissolution or dispersion in a liquid medium.

In a particular embodiment, the ingredients of the tablet according to the present invention: zonisamide, particles comprising a) a sugar or a sugar alcohol, preferably mannitol, b) a cellulose, preferably microcrystalline cellulose, c) an acid-type carboxymethylcellulose as the first disintegrant component, preferably carmellose and d) a second disintegrant component, which is not an acid-type carboxymethylcellulose, preferably crospovidone, a lubricant and optionally one or more sweeteners, are homogeneously mixed together to provide a homogeneous mixture. The mixture is then subjected to direct compression to provide a solid preparation in the form of a tablet. Direct compression method is preferred because it is simpler, easier to automate and avoids direct contact of water with the active material and excipients.

For example, the powder mixture is fed to the die of a manual tablet press, such as a manual IR press, and sufficient pressure is applied to form the solid tablet. Such pressure can vary, and typically ranges from 5 to 200 kN, applied during a time from about 10 to 30 seconds when a manual IR press is used.

In a preferred embodiment of the present invention the pressure applied with the manual IR press to the obtained ingredients' mixture is between 10 to 50 kN during about 10 to 30 seconds, more preferably from 15 to 30 kN during about 10 to 30 seconds, even more preferably from 15 to 20 kN during about 10 to 30 seconds.

In another example, the powder mixture is fed to an automatic rotary tablet press, applying pressure in the range of 5 kN to 100 kN depending on the selected manufacturing speed and on the pressure working limit of the punches to be used. In a preferred embodiment, the pressure will be from 5 kN to 50 kN, preferably 10 kN to 50 kN, preferably from 13 kN to 40 kN, more preferably from 13 kN to 35 kN, and even more preferably from 13 kN to 30 kN. For example, the rotary tablet press Ronchi FA TOP 5+5 is able to work with pressures from 13 kN to 25 kN. The skilled person knows such rotary tablet presses and is able to adjust the required pressure so that for example the pressure on the punches does not exceed their pressure working limit.

Direct compression is the easiest way of manufacturing tablets and has the great advantage of having a low manufacturing cost. Moreover, it uses conventional equipment, commonly available excipients and a limited number of process steps.

The resulting compressed solid preparation possesses a suitable strength and hardness and does not disintegrate during distribution and storage.

In another embodiment the present invention relates to the orodispersible tablet formulation for use in the treatment of epilepsy, and Parkinson's disease.

In a third aspect, the orally disintegrating tablets of the present invention prepared by direct compression may be conditioned in a packaging material such as aluminium / aluminium (Alu/Alu), polypropylene / aluminium (PP/Alu), polyvinyl chloride / aluminium (PVC/Alu), polyvinylidene dichloride / aluminium (PVDC/Alu), polyvinyl chloride / polyvinylidene dichloride / aluminium (PVC/PVDC/Alu), polyvinyl chloride / polypropylene / polyvinylidene dichloride / aluminium (PVC/PE/PVDC/Alu). Suitable polypropylene films have a thickness of from 200 µm to 400 µm, preferably the polypropylene film thickness is of 300 µm. Suitable polyvinyl chloride films have a thickness of from 200 µm to 400 µm, preferably the polyvinyl chloride film thickness is of 250 µm. Suitable polyvinylidene dichloride films grammage are of from 90 g/m² to 180 g/m², preferably the polyvinylidene dichloride film is of 120 g/m². Suitable Alu seal have a thickness of from 20 µm to 30 µm, preferably the Alu seal is of 20 µm.

The orally disintegrating tablets packaged into a film as defined above may be further packaged into an Alu bag together with silica gel as desiccant (e.g. in the form of a sachet or strip) to improve the protection from moisture.

In an embodiment the orally disintegrating tablets are packaged into a blister made of polypropylene / aluminium (PP/Alu), with at least one sachet of silica gel into Alu bag. Preferably, the PP film thickness is of 300 µm and Alu seal thickness is of 20 µm.

In an embodiment the orally disintegrating tablets are packaged in a blister made of PVDC/Alu into Alu bag having at least one sachet of silica gel. Preferably, the PVDC grammage is of 90 g/m² and Alu seal thickness is of 20 µm.

In another preferred embodiment the orally disintegrating tablets of the invention are conditioned in a blister made of PVC/PE/PVDC/Alu. Preferably, the orally disintegrating tablets are packaged in a blister made of PVC/PE/PVDC/Alu, wherein the PVC film thickness is of 250 µm, the PE film thickness is of 25 µm, and the PVDC SUPER B film grammage is of 120 g/m² transparent. The orally disintegrating tablets packaged into a blister of PVC/PE/PVDC SUPER B/Alu have good to excellent storage stability of at least 6 months at 25°C / 60% RH and at 40°C / 75% RH, in other words, tablet hardness, disintegration time, water content and related substances of the orally disintegrating tablets packaged into a blister of PVC/PE/PVDC SUPER B/Alu do not significantly change under these stability conditions.

Thus, in an embodiment the orally disintegrating tablets of the invention are packaged into a polymer film made of polyvinyl chloride, polypropylene and polyvinylidene dichloride, and an aluminium seal, more preferably in a polyvinyl chloride film having a thickness of 250 µm, a polypropylene film having a thickness of 25 µm, a polyvinylidene dichloride film having a grammage of 120 g/m² and an aluminium seal having a thickness of 20 µm, without the need to use Alu bag together with silica gel as desiccant in the form of sachet or strip. Advantageously, the package film made of PVC/PE/PVDC SUPER B/Alu protects enough the orally disintegrating tablets of the invention from moisture, oxygen, gas or vapour during the patient use (e.g. for 30 days of the treatment dosage) without the need to protect them in a specially manner compared to commercially available tablets. Moreover, the fact that the packaging is transparent allows the patient to differentiate between different strengths (i.e. 25 mg and 50 mg).

The following non-limiting examples will further illustrate specific embodiments of the invention. They are, however, not intended to be limiting the scope of the present invention in any way.

### Examples

### General manufacturing method for the orodispersible tablets

The orodispersible tablets were obtained according to the following procedure:
i. the components (i) to (iv) of the formulation were weighed;
i. zonisamide and particles (ii) were mixed together;
ii. optionally, component (iv) was sieved through a screen with a mesh size of 250 µm, while being added to the mixture of step ii);
iii. the resulting mixture was further mixed for 15 minutes;
iv. component (iii) was sieved through a screen with a mesh size of 250 µm, while being added to the mixture of step iv);
v. the resulting mixture was further mixed for 5 minutes to ensure homogeneity;
vi. the obtained mixture powder was compressed in a tabletting machine equipped with suitable punches.

For the preparation of the herein provided examples, regarding the particles comprising mannitol, microcrystalline cellulose, carmellose and crospovidone, *i.e.* component (ii), the commercially available product Granfiller-D^{®} 215 (from Daicel) was used. Said product is available in the form of granules, having a median particle size of 140 µm, having the following composition.

| Components | Amount (in weight percentage) |
|---|---|
| D-Mannitol | 53.0% - 59.0% |
| Microcrystalline cellulose | 17.0% - 23.0 % |
| Carmellose | 15.0% - 23.0 % |
| Crospovidone | 3.4 % - 4.6 % |

### Tableting machines

The tableting machines used for the preparation of the tablets of the examples were:
- a manually operated hydraulic IR press Perkin Elmer Specac 15011, equipped with a round punches of 10 mm of diameter; and
- a rotary press Ronchi FA TOP 5+5, equipped with round, flat-faced bevel-edged punches, which can be of 10.5 mm (for tablets containing 50 mg of zonisamide) or of 8.0 mm (for tablets containing 25 mg of zonisamide) diameter; optionally, the 10.5 mm punch may have a two faces bisect.

### Disintegration time assay

The disintegration times of the formulations according to the present invention were assayed under two different protocols.

### Disintegration protocol I

A tablet according to the present invention is placed in a glass beaker having 65 mm of diameter, containing 40 mL of purified water, at 37.5°C. The time of the onset of disintegration (i = initial), and the time of complete disintegration are recorded. The disintegration time is the time when the tablet completely disintegrates. Generally, 3 samples were tested.

### Disintegration protocol II

A tablet according to the present invention is placed in a manual disintegration apparatus of the type Tablet Disintegration Tester DTG 1000 of Copley Scientific filled with 800 mL of purified water, at 37.5 °C. The disintegration time is the time when the tablet completely disintegrates. Generally, 6 samples were tested.

### Hardness test (Resistance to crushing)

Tablet hardness is tested according to Ph. Eur. 2.9.8. Depending on the batch size, 3, 5 or 10 samples are tested.

### KF (water content)

The analyses to determine the water content were performed in a Karl-Fischer Coulometrics Metrohm Coulometer 831 oven 774. The method according to analytical procedure: European Pharmacopeia (2.5.32, monograph), Water: micro determination, is the following:
Grind 5 tablets and from the obtained powder weight, accurately, about 100 mg. Transfer to a vial and determine the content of water with a Karl-Fischer coulometer at a temperature of 150°C (perform in duplicate).

### Particle Size Distribution (PSD)

The determination of the particle size by Laser light diffraction is carried out using a Malvern Mastersizer 2000 particle analyzer.

### Method parameters:

Sample handling unit: Hydro 2000SM
Particle RI: (1.529, 1)
Dispersant RI: 1 .330 (n-heptane)
Dispersant: n-Heptane + 1% soy lecithin
Analysis model: General purpose
Dispersion unit solvent: n-Heptane

### Samples preparation

Transfer about 50 mg of sample to a recipient of 50 mL, add about 10 mL of n-heptane + 1% soy lecithin and stir the resulting mixture until homogenization.

### Execution

Rinse the accessory Hydro 2000SM with heptane for at least 2 times. Recirculation pumping is about 2500 rpm. Perform an automatic alignment and once the alignment displayed is stable, obtain a blank lecture with heptane and correct. Place a suitable amount of sample, with the aid of a pipette, to obtain a good obscuration (between 10 - 20%). As soon as the desired obscuration is reached, perform the sample measurement, analyse the data and display the result.

### Sample number and tablet weight

20 tablets were weighed in an automatic balance and the average mass was calculated.

Orodispersible tablets were made according to the general method defined above using the ingredients shown in tables 1 and 2. For examples 1-6 and 8, the compression force applied was 20 kN during about 10 seconds using the manual IR press, exception is only made for example 4, wherein the compression force applied was 18 kN during about 10 seconds using the manual IR press. Example 7 was manufactured using the rotary press Ronchi FA TOP 5+5 using a compression force of about 15 kN. The PSD of zonisamide used in examples 1-6 and 8 was characterized by a D(97) of 46 µm measured by volume and a D(90) of 33 µm measured by volume. The PSD of zonisamide used in example 7 was characterized by a D(97) of 41 µm measured by volume and by a D(90) of 31 µm measured by volume.

**Table 1**

| Component | **Example 1** | | **Example 2** | | **Example 3*** | |
|---|---|---|---|---|---|---|
| | Weight (mg) | % (w/w) | Weight (mg) | % (w/w) | Weight (mg) | % (w/w) |
| (i). Zonisamide | 50.0 | 12.50 | 50.0 | 12.50 | 50.0 | 12.5 |
| (ii) Granfiller-D^{®} 215 | 346.0 | 86.5 | 346.0 | 86.5 | 326.0 | 81.5 |
| (iii) Magnesium stearate | 4.0 | 1 | ---- | ---- | ---- | ---- |
| (iii) Sodium stearyl fumarate (Pruv^{®}) | ---- | ---- | 4.0 | 1 | 4.0 | 1 |
| (iv) Aspartame powder Ajinomoto | ---- | ----- | ---- | ----- | 20.0 | 5 |
| (iv) Aspartame Hyet | ---- | ----- | ---- | ----- | ---- | ----- |
| (iv) Aspartame Sinosweet | ---- | ----- | ---- | ----- | ---- | ----- |
| Total | 400 | 100 | 400 | 100 | 400 | 100 |

| | | | | | | |
|---|---|---|---|---|---|---|
| * the force used for this tablet was 1800 tones | | | | | | |

**Table 2**

| Component | **Example 4** | | **Example 5** | | **Example 6** | |
|---|---|---|---|---|---|---|
| | Weight (mg) | % (w/w) | Weight (mg) | % (w/w) | Weight (mg) | % (w/w) |
| (i). Zonisamide | 50.0 | 12.5 | 50.0 | 12.5 | 50.0 | 12.5 |
| (iia) Granfiller-D^{®} 215 | 336.0 | 84 | 336.0 | 84 | 336.0 | 84 |
| (iii) Magnesium stearate | ---- | ---- | ---- | ---- | ---- | ---- |
| (iii) Sodium stearyl fumarate (Pruv^{®}) | 4.0 | 1 | 4.0 | 1 | 4.0 | 1 |
| (iv) Aspartame powder Ajinomoto | 10 | 2.5 | ---- | ----- | ---- | ----- |
| (iv) Aspartame Hyet | ---- | ----- | 10 | 2.5 | ---- | ----- |
| (iv) Aspartame Sinosweet | ---- | ----- | ---- | ----- | 10 | 2.5 |
| Total | 400 | 100 | 400 | 100 | 400 | 100 |

**Table 3**

| Component | **Example 7** | | **Example 8** | |
|---|---|---|---|---|
| | Weight (mg) | % (w/w) | Weight (mg) | % (w/w) |
| (i). Zonisamide | 25.0 | 12.5 | 50.0 | 12.5 |
| (iia) Granfiller-D^{®} 215 | 168.0 | 84 | ---- | ---- |
| (iib) Granfiller-D^{®} 211 | ---- | ---- | 346.0 | 86.5 |
| (iii) Magnesium stearate | ---- | ---- | 4.0 | 1 |
| (iii) Sodium stearyl fumarate (Pruv^{®}) | 2.0 | 1 | ---- | ---- |
| (iv) Aspartame powder Ajinomoto | ---- | ---- | ---- | ---- |
| (iv) Aspartame Hyet | 5.0 | 2.5 | ---- | ---- |
| (iv) Aspartame Sinosweet | ---- | ----- | ---- | ----- |
| Total | 200 | 100 | 400 | 100 |

The hardness and disintegration times of the tablets of the examples are shown in the table 4 below.

**Table 4**

| **Example** | **Hardness** | **Disintegration time protocol *I*** (s) | **Disintegration time protocol *II*** (s) |
|---|---|---|---|
| **1** | 134.0 | 18 (i=7) | 17 |
| **2** | 148.6 | 22 (i=10) | 17 |
| **3** | 156.6 | 19 (i=11 s) | 20 |
| **4** | 143.9* | 16 (i=2) | 20 |
| **5** | 159.6 | 16 (i=5) | 17 |
| **6** | 161.1 | 18 (i=2) | 19 |
| **7** | 95.2 | 21 (i=12) | 22 |
| **8** | 145.2 | 30 | 25 |
| **Trerief^{®} 50 mg** | 114 | 33 (i=2) | 29 |
| **Trerief^{®} 25 mg** | 67.5 | 22(i=4) | 28 |

| | | | |
|---|---|---|---|
| * the force used for this tablet was 1800 tones i = initial → represents the time in seconds when disintegration starts | | | |

### Comparative example 1

The properties of the tablet formulations according to the invention, (example 4) were compared with a commercially available ODT formulation comprising zonisamide 50 mg (Trerief^{®} OD Tablets 50 mg). Their hardness, and respective disintegration times were measured at day 0 of the experiment and after 15 days storage, at 25°C / 75% RH and 40°C / 75% relative humidity (RH) under unpackaged conditions:

**Table 5**

| Parameter | ODT Formulation (50 mg Zonisamide) | | | | | |
|---|---|---|---|---|---|---|
| | **Example 4** | | | Trerief^{®} | | |
| | Day 0 | Day 15 | | Day 0 | Day 15 | |
| | | 25°C /75% RH | 40°C /75% RH | | 25°C /75% RH | 40°C /75% RH |
| Tablet weight (mg) | 397.9 | 407.8 | 404.3 | 400.46 | 403.9 | 403.3 |
| Hardness (N) | 143.9 | 76.9 | 79.4 | 114.1 | 56.6 | 56.7 |
| Loss of hardness | NA | 46.6% | 44.8% | NA | 50.4% | 50.3% |
| Disintegration time *I* (s) | 16 | 15 | 17 | 33 | 28 | 30 |
| Disintegration time *II* (s) | 20 | 11 | 13 | 29 | 21 | 21 |

As can be seen from table 5, the tablets obtained according to example 4 of the present invention, display a higher tablet hardness both at day 0 and after 15 days under accelerated storage conditions (i.e. 40°C / 75% RH). The resulting loss of hardness is less than 50% in case of example 4, while Trerief^{®} shows a loss of hardness of more than 50%. Furthermore, even though the tablets of the present invention have higher hardness, the dissolution times are always 25 seconds or less, and shorter when compared with Trerief^{®}. In some examples, the disintegration times are even less than 20 seconds.

Thus, the tablets according to the present invention display a higher tablet hardness than the commercially available formulation, whilst maintaining or reducing the tablets dissolution times.

### Stability studies

Orally disintegrating tablets of 25 mg and 50 mg strengths of zonisamide were prepared according to compositions of the examples 7 and 5, respectively, using the rotary press Ronchi FA TOP 5+5. The so-prepared samples were stored under unpackaged conditions at 25 °C / 75% RH for 1 month, 2 months and 3 months. The PSD of zonisamide used was characterized by a D(97) of 107 µm and by a D(90) of 48 µm measured by volume. The tablet hardness and disintegration times were measured as defined above and gathered in tables 6 and 7.

**Table 6**

| **Parameter** | ODT (25 mg Zonisamide) at 25 °C / 75% RH | | | |
|---|---|---|---|---|
| Time | Day 0 | 1 month | 2 months | 3 months |
| Tablet weight (mg) | 197.5 | 208.3 | 207.1 | 207.5 |
| Hardness (N) | 103.9 | 59.2 | 63.3 | 65.4 |
| Disintegration time I(s) | 19.7 | 12.0 | 13.0 | 12.3 |
| Disintegration time II (s) | 12.7 | 8.5 | 8.2 | 17.0 |
| KF | 3.0 | 5.2 | 4.95 | 5.0 |

**Table 7**

| **Parameter** | ODT (50 mg Zonisamide) at 25 °C / 75% RH | | | |
|---|---|---|---|---|
| Time | Day 0 | 1 month | 2 months | 3 months |
| Tablet weight (mg) | 395.0 | 410.5 | 406.9 | 409.7 |
| Hardness (N) | 150.2 | 85.1 | 90.9 | 93.9 |
| Disintegration time I(s) | 23.0 | 19.0 | 14.3 | 18.7 |
| Disintegration time II (s) | 16.0 | 11.0 | 10.0 | 13.5 |
| KF | 2.9 | 5.2 | 4.92 | 5.2 |

According to tables 6 and 7, the orally disintegrating tablets maintain more than 50% of their initial hardness upon storage conditions at 25 °C / 75% RH for at least 3 months while the disintegration times are less than 20 s. Noteworthy, the small increase in the amount of water content generally observed after storage conditions do not affect significantly the hardness of the tablets and, despite this high hardness, maintains good to excellent disintegration times after storage conditions at 25 °C / 75% RH for at least 3 months.

### Packaging stability study

Orally disintegrating tablets of 25 mg and 50 mg strengths of zonisamide were prepared according to the compositions of examples 7 and 5, respectively. The so-prepared samples were packaged in a blister made of PVC/PE/PVDC/Alu and stored under stability conditions at 25°C / 60% RH and 40°C / 75% RH for 1 month and 3 months. Tablet hardness and disintegration times were measured as defined above and gathered in table 8.

**Table 8**

| | ODT Formulation (25 mg Zonisamide) | | | | |
|---|---|---|---|---|---|
| Parameter | | 25°C/60%RH | | 40°C/75%RH | |
| Time | Day 0 | 1 month | 3 months | 1 month | 3 months |
| Hardness (N) | 103.8 | 106.6 | 109.4 | 103.4 | 99.0 |
| Dissintegration time I (s) | NA | 23.3 | 20.0 | 17.7 | 18.0 |
| Dissintegration time II (s) | 15.0 | 16.0 | 16.0 | 12.0 | 16.0 |
| Water content % | NA | 3.43 | 3.27 | 3.89 | 3.44 |

| | ODT Formulation (50 mg Zonisamide) | | | | |
|---|---|---|---|---|---|
| Hardness (N) | 144.2 | 163.5 | 156.0 | 163.9 | 158.1 |
| Dissintegration time I (s) | 31.7 | 26.0 | 25.5 | 23.7 | 23.0 |
| Dissintegration time II (s) | 22.9 | 20.0 | 17.0 | 16.2 | 20.0 |
| Water content % | 3.96 | 3.36 | 3.18 | 3.37 | 3.48 |

PVC film thickness of 250 µm, PE film thickness of 25 µm, and PVDC SUPER B film grammage of 120 g/m² transparent and aluminium seal thickness of 20 µm were used.

The orally disintegrating tablets packaged into a blister of PVC/PE/PVDC/Alu have good to excellent storage stability at 25°C / 60% RH and at 40°C / 75% RH for at least 3 months. Moreover, the ODT formulations which contain 25 mg of zonisamide have a hardness of more than 98 N and a disintegration time, measured according to Disintegration protocol II, of less than 30 seconds and, after storage stability at 25°C / 60% RH and/or at 40°C / 75% RH for at least one month, preferably for at least three months. The ODT formulations which contain 50 mg of zonisamide have a hardness of more than 100 N and a disintegration time, measured according to Disintegration protocol II, of less than 30 seconds and, after storage stability at 25°C / 60% RH and/or at 40°C / 75% RH for at least one month, preferably for at least three months.

## Claims

1. An orally disintegrating tablet prepared by direct compression comprising:
(i) zonisamide,
(ii) particles comprising:
a) mannitol,
b) microcrystalline cellulose;
c) carmellose;
d) crospovidone;
(iii) a lubricant;
(iv) optionally, one or more sweeteners.

2. The orally disintegrating tablet according to claim 1, wherein the percentage of zonisamide or a pharmaceutically acceptable salt thereof is of from 1% to 20% by weight of the tablet.

3. The orally disintegrating tablet according to any one of the preceding claims, comprising from 80% to 90% by weight of the tablet of particles (ii), wherein the particles (ii) preferably comprise (a) from 40% to 60% by weight of mannitol by weight of the particles, (b) from 10% to 30% of microcrystalline cellulose by weight of the particles, (c) from 10% to 30% of carmellose by weight of the particles and (d) from 1% to 7% of crospovidone by weight of the particles.

4. The tablet according to any one of the preceding claims, wherein the weight percentage of the lubricant is from 0.1% to 5% by weight of the tablet and wherein the component iii) is preferably selected from the group comprising: talc, sodium benzoate, sodium stearyl fumarate (Pruv^{®}), calcium stearate, magnesium stearate, zinc stearate, glyceryl behenate, stearic acid and glyceryl monostearate, or mixtures thereof, more preferably the component iii) is sodium stearyl fumarate (Pruv^{®}).

5. The tablet according to any one of the preceding claims, wherein the weight percentage of the optional one or more sweeteners is from 0.1% to 7% by weight of the tablet.

6. The tablet according to anyone of the preceding claims, wherein particles of zonisamide i) have a particle size distribution **characterised by** a D(97) in the range of 30 µm to 300 µm measured by volume, preferably a D(97) equal or less than 200 µm measured by volume, more preferably D(97) equal or less than 150 µm measured by volume.

7. An orally disintegrating tablet according to claims 1 to 6, which:
a) contain 50 mg of zonisamide and have a hardness of more than 100 N and a disintegration time, measured according to Disintegration protocol II, of less than 30 seconds; or
b) contain 25 mg of zonisamide and have a hardness of more than 80 N and a disintegration time, measured according to Disintegration protocol II, of less than 30 seconds.

8. An orally disintegrating tablet according to claims 1 to 7, which:
a) contain 50 mg of zonisamide and have a hardness of more than 65 N and a disintegration time, measured according to Disintegration protocol II, of less than 30 seconds after storage stability at 25°C / 75% RH for at least one month, preferably for at least two months, more preferably for at least three months; or
b) contain 25 mg of zonisamide and have a hardness of more than 40 N and a disintegration time, measured according to Disintegration protocol II, of less than 30 seconds after storage stability at 25°C / 75% RH for at least one month, preferably for at least two months, more preferably for at least three months.

9. An orally disintegrating tablet according to claims 1 to 8, which:
a) contain 50 mg of zonisamide and maintain more than 50% of their initial hardness and a disintegration time, measured according to Disintegration protocol II, of less than 30 seconds after storage stability at 25°C / 75% RH for at least one month, preferably for at least two months, more preferably for at least three months; or
b) contain 25 mg of zonisamide and maintain more than 50% of their initial hardness and a disintegration time, measured according to Disintegration protocol II, of less than 30 seconds after storage stability at 25°C / 75% RH for at least one month, preferably for at least two months, more preferably for at least three months.

10. The orally disintegrating tablet according to any one of the preceding claims for use in the treatment of epilepsy, and Parkinson's disease.

11. A process for the preparation of an orally disintegrating tablet as defined in anyone of claims 1 to 8, comprising:
a) preparing a mixture comprising at least the ingredients (i), (ii), (iii) and optionally (iv), and;
b) applying direct compression to the mixture obtained in step a).

12. The process according to claim 11, wherein the pressure applied in step b) is between 5 to 50 kN.

13. An orally disintegrating tablet as defined in anyone of claims 1 to 7, packaged into a polymer film made of polyvinyl chloride, polypropylene and polyvinylidene dichloride, and an aluminium seal, wherein the polyvinyl chloride film has preferably a thickness of 250 µm, the polypropylene film has preferably a thickness of 25 µm, the polyvinylidene dichloride film grammage is preferably of 120 g/m² and the aluminium seal has preferably a thickness of 20 µm.

14. The orally disintegrating tablet according to claim 13, comprising 50 mg of zonisamide, which:
a) have a hardness of more than 100 N and a disintegration time, measured according to Disintegration protocol II, of less than 30 seconds after storage stability at 25°C / 60% RH for at least one month, preferably for at least three months; or
b) have a hardness of more than 100 N and a disintegration time, measured according to Disintegration protocol II, of less than 30 seconds after storage stability at 40°C / 75% RH for at least one month, preferably for at least three months.

15. The orally disintegrating tablet according to claim 13, comprising 25 mg of zonisamide, which:
a) have a hardness of more than 100 N and a disintegration time, measured according to Disintegration protocol II, of less than 30 seconds after storage stability at 25°C / 60% RH for at least one month, preferably for three months; or
b) have a hardness of more than 98 N and a disintegration time, measured according to Disintegration protocol II, of less than 30 seconds after storage stability at 40°C / 75% RH for at least one month, preferably for at least three months.

## Patentansprüche

1. Oral zerfallende Tablette, hergestellt durch direkte Kompression, umfassend:
(i) Zonisamid
(ii) Partikel, umfassend:
a) Mannitol,
b) mikrokristalline Cellulose;
c) Carmellose;
d) Crospovidon;
(iii) ein Gleitmittel;
(iv) wahlweise, ein oder mehrere Süßungsmittel.

2. Oral zerfallende Tablette nach Anspruch 1, wobei der prozentuale Anteil an Zonisamid oder eines pharmazeutisch akzeptablen Salzes davon von 1% bis 20%, bezogen auf das Gewicht der Tablette, beträgt.

3. Oral zerfallende Tablette nach einem der vorhergehenden Ansprüche, umfassend von 80% bis 90% an Partikeln (ii), bezogen auf das Gewicht der Tablette, wobei die Partikel (ii) vorzugsweise (a) von 40 Gew.% bis 60 Gew.% Mannitol, bezogen auf das Gewicht der Partikel, (b) von 10% bis 30% mikrokristalliner Cellulose, bezogen auf das Gewicht der Partikel, (c) von 10% bis 30% Carmellose, bezogen auf das Gewicht der Partikel, und (d) von 1% bis 7% Crospovidon, bezogen auf das Gewicht der Partikel, umfassen.

4. Tablette nach einem der vorhergehenden Ansprüche, wobei der gewichtsprozentuale Anteil des Gleitmittels 0,1% bis 5%, bezogen auf das Gewicht der Tablette, beträgt und wobei die Komponente iii) vorzugsweise ausgewählt ist aus der Gruppe, umfassend: Talkum, Natriumbenzoat, Natriumstearylfumarat (Pruv^{®}), Calciumstearat, Magnesiumstearat, Zinkstearat, Glycerylbehenat, Stearinsäure und Glycerylmonostearat, oder Mischungen davon, wobei bevorzugter die Komponente iii) Natriumstearylfumarat (Pruv^{®}) ist.

5. Tablette nach einem der vorhergehenden Ansprüche, wobei der gewichtsprozentuale Anteil der wahlweisen einen oder mehrerer Süßungsmittel von 0,1% bis 7%, bezogen auf das Gewicht der Tablette, beträgt.

6. Tablette nach einem der vorhergehenden Ansprüche, wobei die Partikel des Zonisamids i) eine Partikelgrößenverteilung haben, die durch einen D(97) im Bereich von 30 µm bis 300 µm, nach Volumen gemessen, gekennzeichnet ist, vorzugsweise einen D(97) gleich oder weniger als 200 µm, nach Volumen gemessen, noch bevorzugter einen D(97) gleich oder weniger als 150 µm, nach Volumen gemessen.

7. Oral zerfallende Tablette nach den Ansprüchen 1 bis 6, die:
a) 50 mg Zonisamid enthält und eine Härte von mehr als 100 N und eine Zerfallszeit, gemessen nach dem Zerfallsprotokoll II, von weniger als 30 Sekunden hat; oder
b) 25 mg Zonisamid enthält und eine Härte von mehr als 80 N und eine Zerfallszeit, gemessen nach dem Zerfallsprotokoll II, von weniger als 30 Sekunden hat.

8. Oral zerfallende Tablette nach den Ansprüchen 1 bis 7, die:
a) 50 mg Zonisamid enthält und eine Härte von mehr als 65 N und eine Zerfallszeit, gemessen nach dem Zerfallsprotokoll II, von weniger als 30 Sekunden hat, nach Lagerstabilität bei 25°C / 75% RH für mindestens einen Monat, vorzugsweise für mindestens zwei Monate, bevorzugter für mindestens drei Monate; oder
b) 25 mg Zonisamid enthält und eine Härte von mehr als 40 N und eine Zerfallszeit, gemessen nach dem Zerfallsprotokoll II, von weniger als 30 Sekunden hat, nach Lagerstabilität bei 25°C / 75% RH für mindestens einen Monat, vorzugsweise für mindestens zwei Monate, bevorzugter für mindestens drei Monate.

9. Oral zerfallende Tablette nach den Ansprüchen 1 bis 8, die:
a) 50 mg Zonisamid enthält und mehr als 50% ihrer anfänglichen Härte und eine Zerfallszeit, gemessen nach dem Zerfallsprotokoll II, von weniger als 30 Sekunden beibehält, nach Lagerstabilität bei 25°C / 75% RH für mindestens einen Monat, vorzugsweise für mindestens zwei Monate, bevorzugter für mindestens drei Monate; oder
b) 25 mg Zonisamid enthält und mehr als 50% ihrer ursprünglichen Härte und eine Zerfallszeit, gemessen nach dem Zerfallsprotokoll II, von weniger als 30 Sekunden beibehält, nach Lagerstabilität bei 25°C / 75% RH für mindestens einen Monat, vorzugsweise für mindestens zwei Monate, bevorzugter für mindestens drei Monate.

10. Oral zerfallende Tablette nach einem der vorhergehenden Ansprüche zur Verwendung bei der Behandlung von Epilepsie und der Parkinson-Krankheit.

11. Verfahren zur Herstellung einer oral zerfallenden Tablette, wie in einem der Ansprüche 1 bis 8 definiert, umfassend:
a) Herstellen einer Mischung, die mindestens die Bestandteile (i), (ii), (iii) und wahlweise (iv) umfasst, und;
b) Anwendung direkter Kompression auf die in Schritt a) erhaltene Mischung.

12. Verfahren nach Anspruch 11, wobei der in Schritt b) aufgebrachte Druck zwischen 5 und 50 kN beträgt.

13. Oral zerfallende Tablette, wie in einem der Ansprüche 1 bis 7 definiert, verpackt in einen Polymerfilm, der aus Polyvinylchlorid, Polypropylen und Polyvinylidendichlorid und einer Aluminiumversiegelung gemacht ist, wobei der Polyvinylchloridfilm vorzugsweise eine Dicke von 250 µm hat, der Polypropylenfilm vorzugsweise eine Dicke von 25 µm hat, das Flächengewicht des Polyvinylidendichloridfilms vorzugsweise 120 g/m² beträgt und die Aluminiumversiegelung vorzugsweise eine Dicke von 20 µm hat.

14. Oral zerfallende Tablette nach Anspruch 13, umfassend 50 mg Zonisamid, die:
a) eine Härte von mehr als 100 N und eine Zerfallszeit, gemessen nach dem Zerfallsprotokoll II, von weniger als 30 Sekunden hat, nach Lagerstabilität bei 25°C / 60% RH für mindestens einen Monat, vorzugsweise für mindestens drei Monate; oder
b) eine Härte von mehr als 100 N und eine Zerfallszeit, gemessen nach dem Zerfallsprotokoll II, von weniger als 30 Sekunden hat, nach Lagerstabilität bei 40°C / 75% RH für mindestens einen Monat, vorzugsweise für mindestens drei Monate.

15. Oral zerfallende Tablette nach Anspruch 13, umfassend 25 mg Zonisamid, die:
a) eine Härte von mehr als 100 N und eine Zerfallszeit, gemessen nach dem Zerfallsprotokoll II, von weniger als 30 Sekunden hat, nach Lagerstabilität bei 25°C / 60% RH für mindestens einen Monat, vorzugsweise für drei Monate; oder
b) eine Härte von mehr als 98 N und eine Zerfallszeit, gemessen nach dem Zerfallsprotokoll II, von weniger als 30 Sekunden hat, nach Lagerstabilität bei 40°C / 75% RH für mindestens einen Monat, vorzugsweise für mindestens drei Monate.

## Revendications

1. Un comprimé à désintégration orale préparé par compression directe comprenant :
(i) du zonisamide,
(ii) des particules comprenant :
a) du mannitol,
b) de la cellulose microcristalline ;
c) de la carmellose ;
d) de la crospovidone ;
(iii) un lubrifiant ;
(iv) optionnellement, un ou plusieurs édulcorants.

2. Le comprimé à désintégration orale selon la revendication 1, dans lequel le pourcentage de zonisamide ou d'un sel pharmaceutiquement acceptable de celui-ci est compris entre 1 % et 20 % en poids du comprimé.

3. Le comprimé à désintégration orale selon l'une quelconque des revendications précédentes, comprenant de 80 % à 90 % en poids du comprimé de particules (ii), les particules (ii) comprenant de préférence (a) de 40 % à 60 % en poids de mannitol en poids des particules, (b) de 10 % à 30 % de cellulose microcristalline en poids des particules, (c) de 10 % à 30 % de carmellose en poids des particules et (d) de 1 % à 7 % de crospovidone en poids des particules.

4. Le comprimé selon l'une quelconque des revendications précédentes, dans lequel le pourcentage en poids du lubrifiant est de 0,1% à 5% en poids du comprimé et dans lequel le composant iii) est de préférence choisi dans le groupe comprenant : le talc, le benzoate de sodium, le fumarate de stéaryle de sodium (Pruv^{®}), le stéarate de calcium, le stéarate de magnésium, le stéarate de zinc, le béhénate de glycéryle, l'acide stéarique et le monostéarate de glycéryle, ou des mélanges de ceux-ci, de façon encore préférée le composant iii) est le fumarate de stéaryle de sodium (Pruv^{®}).

5. Le comprimé selon l'une quelconque des revendications précédentes, dans lequel le pourcentage en poids du ou des édulcorants facultatifs est de 0,1 % à 7 % en poids du comprimé.

6. Le comprimé selon l'une quelconque des revendications précédentes, dans lequel les particules de zonisamide i) ont une distribution granulométrique **caractérisée par** un D(97) compris dans la gamme allant de 30 µm à 300 µm mesuré en volume, de préférence un D(97) égal ou inférieur à 200 µm mesuré en volume, de façon encore préférée D(97) égal ou inférieur à 150 µm mesuré en volume.

7. Un comprimé à désintégration orale selon les revendications 1 à 6, qui :
a) contient 50 mg de zonisamide et a une dureté supérieure à 100 N et un temps de désintégration, mesuré selon le protocole de Désintégration II, inférieur à 30 secondes ; ou
b) contient 25 mg de zonisamide et a une dureté supérieure à 80 N et un temps de désintégration, mesuré selon le protocole de Désintégration II, inférieur à 30 secondes.

8. Un comprimé à désintégration orale selon les revendications 1 à 7, qui :
a) contient 50 mg de zonisamide et a une dureté supérieure à 65 N et un temps de désintégration, mesuré selon le protocole de Désintégration II, inférieur à 30 secondes après stabilité au stockage à 25°C / 75 % HR pendant au moins un mois, de préférence pendant au moins deux mois, de façon encore préférée pendant au moins trois mois ; ou
b) contient 25 mg de zonisamide et a une dureté supérieure à 40 N et un temps de désintégration, mesuré selon le protocole de Désintégration II, inférieur à 30 secondes après stabilité au stockage à 25°C / 75 % HR pendant au moins un mois, de préférence pendant au moins deux mois, de façon encore préférée pendant au moins trois mois.

9. Le comprimé à désintégration orale selon les revendications 1 à 8, qui :
a) contient 50 mg de zonisamide et conserve plus de 50 % de leur dureté initiale et un temps de désintégration, mesuré selon le protocole de Désintégration II, inférieur à 30 secondes après stabilité au stockage à 25°C / 75 % HR pendant au moins un mois, de préférence pendant au moins deux mois, de façon encore préférée pendant au moins trois mois ; ou
b) contient 25 mg de zonisamide et conserve plus de 50 % de leur dureté initiale et un temps de désintégration, mesuré selon le protocole de Désintégration II, inférieur à 30 secondes après stabilité au stockage à 25°C / 75 % HR pendant au moins un mois, de préférence pendant au moins deux mois, de façon encore préférée pendant au moins trois mois.

10. Le comprimé à désintégration orale selon l'une quelconque des revendications précédentes destiné à être utilisé dans le traitement de l'épilepsie et de la maladie de Parkinson.

11. Un procédé de préparation d'un comprimé à désintégration orale tel que défini dans l'une quelconque des revendications 1 à 8, comprenant :
a) le fait de préparer un mélange comprenant au moins les ingrédients (i), (ii), (iii) et optionnellement (iv), et ;
b) le fait d'appliquer une compression directe au mélange obtenu à l'étape a).

12. Le procédé selon la revendication 11, dans lequel la pression appliquée à l'étape b) est comprise entre 5 et 50 kN.

13. Un comprimé à désintégration orale tel que défini dans l'une quelconque des revendications 1 à 7, conditionné dans un film polymère constitué de chlorure de polyvinyle, de polypropylène et de dichlorure de polyvinylidène, et un joint en aluminium, le film de chlorure de polyvinyle ayant de préférence une épaisseur de 250 µm, le film de polypropylène a de préférence une épaisseur de 25 µm, le grammage du film de dichlorure de polyvinylidène est de préférence de 120 g/m² et le joint en aluminium a de préférence une épaisseur de 20 µm.

14. Le comprimé à désintégration orale selon la revendication 13, comprenant 50 mg de zonisamide, qui :
a) a une dureté supérieure à 100 N et un temps de désintégration, mesuré selon le protocole de Désintégration II, inférieur à 30 secondes après stabilité au stockage à 25°C / 60 % HR pendant au moins un mois, de préférence pendant au moins trois mois ; ou
b) a une dureté supérieure à 100 N et un temps de désintégration, mesuré selon le protocole de Désintégration II, inférieur à 30 secondes après stabilité au stockage à 40°C / 75% HR pendant au moins un mois, de préférence pendant au moins trois mois.

15. Le comprimé à désintégration orale selon la revendication 13, comprenant 25 mg de zonisamide, qui :
a) a une dureté supérieure à 100 N et un temps de désintégration, mesuré selon le protocole de Désintégration II, inférieur à 30 secondes après stabilité au stockage à 25°C / 60 % HR pendant au moins un mois, de préférence pendant trois mois ; ou
b) a une dureté supérieure à 98 N et un temps de désintégration, mesuré selon le protocole de Désintégration II, inférieur à 30 secondes après stockage à 40°C / 75% HR pendant au moins un mois, de préférence pendant au moins trois mois.
